Europäisches Patentamt

European Patent Office

Office européen des brevets

(11) Publication number: 0 581 002 A2

# EUROPEAN PATENT APPLICATION

(21) Application number: 93109404.9

(51) Int. Cl.5: C12N 9/50

(22) Date of filing: 11.06.93

(30) Priority: 30.07.92 US 921666

(43) Date of publication of application:
02.02.94 Bulletin 94/05

(84) Designated Contracting States:
AT BE CH DE DK ES FR GB GR IE IT LI LU MC NL PT SE

(71) Applicant: AMERICAN CYANAMID COMPANY
1937 West Main Street
P.O. Box 60
Stamford Connecticut 06904-0060(US)

(72) Inventor: Cibulskas, Algird S.
28 Westwood Road
Stamford, Connecticut 06902(US)
Inventor: Asbell, Henri R.
51 Gilrix Drive
Martinez, California 94553(US)

(74) Representative: Wächtershäuser, Günter, Dr.
Tal 29
D-80331 München (DE)

(54) Use of blends of mannich acrylamide polymers and quaternary polyamines for flocculating enzyme broth streams.

(57) Blends of Mannich acrylamide polymers and quaternary polyamines have been found to be superior flocculants for enzyme broth streams yielding higher solids compaction and greater supernatant clarities than the use of either polymer alone.

EP 0 581 002 A2

Rank Xerox (UK) Business Services
(3.10/3.09/3.3.4)

## BACKGROUND OF THE INVENTION

The production of enzymes by fermentation has been carried out for many years. Fermentation is usually carried out in stainless steel equipment i.e. mixing and blending tanks, and seed and main fermentators. Constant temperature, automatic foam and pH controllers and air purifiers are employed since the absence of foreign microorganisms is essential. Tap water is generally combined with the media ingredients and enzyme recovery begins as soon as fermentation is terminated. The medium is cooled and centrifuges are used to remove bacteria and large insolubles from the supernatant followed by filters to separate smaller particles. The enzymes are concentrated and removed from the filtrate by the addition of a precipitating agent. The precipitate is then further treated by additional filtering and drying etc. and is then standardized such as by using sodium chloride.

Proteases are enzymes which have been found to be particularly useful in industrial areas including cheese making, meat tenderizing, bread baking, beer haze elimination, digestive aid preparations, garment cleaning, pharmaceutical preparation and the like. Those proteases produced by cultivation can be used as food additives.

Characteristic of the protease enzyme broth is the formation of a suspension that does not settle. Upon centrifugation of a sample in a test tube, solids will be deposited in the lower 70% of the test tube and only the upper 30% of the tube will be supernatant solution.

One of the most difficult problems involving enzyme production is the isolation of the enzyme from its broth. Although many flocculating agents have been used for the precipitation of enzyme broths, most have suffered from some disability which renders the agent less attractive commercially. Examples of flocculants used commercially include epichlorohydrin-dimethylamine condensation products cross-linked with diethylenetriamine/dicyanamide; and Mannich acrylamide polymers and polydimethyldiallylammonium halide blends as taught in United States Patent No. 4,997,759. These additives, although useful in many situations, have not proven to be universally acceptable for all enzyme broth clarifications and therefore industry is continually searching for additional materials which are useful for this purpose.

## SUMMARY OF THE INVENTION

The present invention relates to compositions and processes for precipitating aqueous enzyme broths comprising using, as the flocculating agent, blends of a Mannich acrylamide polymer and a quaternary polyamine having a molecular weight of at least about 25,000. Such floccuating agents have been found to provide effective flocculation of precipitate.

## DESCRIPTION OF THE INVENTION INCLUDING PREFERRED EMBODIMENTS

This invention relates to a composition and process for precipitating an aqueous enzyme broth which comprises adding to said broth a flocculant comprising a blend of (1) a Mannich acrylamide polymer and (2) a quaternary polyamine having a molecular weight of at least about 25,000.

The blends are comprised of the two polymers (1) and (2) in a ratio of 99:1 to 1:99, preferably 90:10 to 10:90, by weight, real polymer solids, respectively.

The Mannich acrylamide polymers are generally well known in the art, examples thereof being disclosed in U.S. Pat. No. 4,137,164. Generally, these polymers are homopolymers of acrylamide or copolymers thereof with such commoners as acrylonitrile, methacrylamide, acrylic acid etc. in amounts up to about 50%, preferably 5-50% of the resultant copolymer. The polymers have molecular weights ranging from about 10,000 to about 3,000,000 and are chemically modified to provide dimethylaminomethyl groups to the extent that the polymer contains 25-100 mol percent of these groups, preferably at least 40 mol percent.

The quaternary polyamine can be any polyamine that is commercially available, or it can be prepared by methods known in the prior art. Preferably the quaternary polyamine has a molecular weight in the range of 50,000 to 350,000, especially preferably between 250,000 to 300,000. The preferred quaternary polyamines can be prepared by reacting one mole of a mono(or di-)alkylamine or mono(or di-)alkanol amine, with from about 0.5 to about 1.5 moles, preferably from about 0.7 to about 1.2 moles, of an epihalohydrin at a temperature ranging from about 0° to 100°C. Small amounts, i.e., up to about 10 mole percent of a multifunctional amine may also be coreacted. The foregoing is in accordance with the process described in United States Patent No. 4,717,550, and references cited therein, especially United States Patent Nos. 1,977,253 and 3,248,353. Suitable amines are ammonia, methylamine, dimethylamine, methanolamine, dimethanolamine, ethylenediamine, diethylenetriamine, phenylene diamine and the like, or

2

a mixture of any of the foregoing. Prefered is a mixture comprising dimethylamine and ethylenediamine.

Preferred quaternary polyamines are reaction products of (i) a mono(or di-)alkylamine or mono(or di-) alkanolamine and (ii) epihalohydrin and (iii) up to about 10 mole percent of an alkylenediamine or ammonia. Preferred for the monoalkylamine is monomethylamine, preferred for the monoalkanolamine is monoethanolamine, preferred for the dialkylamine is dimethylamine and preferred for the epihalohydrin is epichlorohydrin.

The most preferred quaternary polyamine is the reaction product of about 50 mole percent of epichlorohydrin, about 49 mole percent of dimethylamine and about 1 mole percent of ethylenediamine. Excellent results are obtained if the resulting polymer has a molecular weight from about 250,000 to about 300,000.

When mixing the polymer composition, the Mannich acrylamide polymer is preferably an aqueous solution with 3-8 percent solids and the quaternary polyamine preferably is an aqueous solution with 50 weight percent solids.

The polymer blend may be added to the enzyme broth as such or the two polymers may be added individually but as near the same time as possible, since the enhanced benefit of the polymers is attributed to their presence in the broth coincidentally. The amount of the blend added to the broth is that effective to produce the clearest supernatant and achieve the highest solid compaction as possible. Generally, amounts ranging from about 10 to 100 grams of polymer blend per liter of broth, preferably from about 25-75 grams per liter, is effective, although higher or lower amounts may be useful in specific instances.

The process of the invention in its preferred embodiments also includes the steps of (b) mixing the enzyme broth and flocculant; and (c) precipitating the enzyme broth/flocculant mixture.

## DESCRIPTION OF THE PREFERRED EMBODIMENTS

The following examples are set forth for purposes of illustration only and are not to be construed as limitations on the present invention. Products A and B are set forth in the following tables, with respect to the amounts employed, as 0.065% aqueous polymer solutions while Products C and D are expressed as a 50.0% aqueous polymer solution. Clarity is measured by UV absorbance at 660 microns, at 550 microns or by visual observation, depending on the sample.

In the following examples, the Mannich acrylamide polymers employed are each Mannich polyacrylamide of 70% aminomethylation and are further designated as follows:

| Product | Percent Solids | Brookfield Viscosity-cps |
|---------|---------------|--------------------------|
| A | 5.9-6.4% | 26,000-34,000 |
| B | 5.5-6.1 | 34,000-46,000 |

The quaternary polyamines are the reaction products of epichlorohydrin, dimethylamine and ethylenediamine and are further designated as follows:

| Product | Percent Solids | Average Molecular Weight |
|---------|---------------|--------------------------|
| C | 49-51% | 300,000 |
| D | 49-51% | 250,000 |

In order to test the effectiveness of various polymers in flocculating enzyme broths, the following test procedure is utilized: To 5 ml of broth in a 15 ml clinical, graduated centrifuge tube are added 5 ml samples of various concentrations of the polymer solutions. Each sample is mixed by inverting the stoppered clinical tube 20 times, the clinical tube is then centrifuged for 5 minutes and the volume of the compacted enzyme is visually measured. The lower the value, the better. In addition, the clarity of the supernatant is measured by UV absorbance at 550 and 660 microns, as the case may be. A value of 0.3-0.4% is acceptable and below 0.1 is superior.

Table I is a measure of the effect of single polymer flocculants on the precipitation of enzyme broths with the UV absorbance measured at 660 microns. It is a comparative table and shows that although Products A, B and/or C individually may perform effectively with regard to compaction (% volume solids) or clarity, the flocculants alone fail to perform satisfactorily as regards both criteria.

## TABLE 1

### EFFECT OF SINGLE POLYMER FLOCCULANTS ON THE FLOCCULATION OF ENZYME BROTH

| Comparative Example No. | Flocculant | g/l Flocculant | Centrifuge 5 Minutes % Volume Solids | Clarity of Supernatant 660 Microns |
|---|---|---|---|---|
| 1* | A | 20 | 14 | WNF** |
| 2* | A | 30 | 17 | 1.04 |
| 3* | A | 40 | 17 | 0.267 |
| 4* | A | 25 | 18 | 0.646 |
| 5* | A | 30 | 16 | 0.349 |
| 6* | A | 35 | 18 | 0.220 |
| 7* | B | 24 | 16 | 0.565 |
| 8* | B | 27 | 17 | 0.455 |
| 9* | B | 33 | 15 | 0.231 |
| 10* | C | 15 | 24 | 0.487 |
| 11* | C | 20 | 23 | 0.466 |
| 12* | C | 25 | 23 | 0.496 |

\* Comparative Example, not in accordance with the invention

\*\* WNF--would not filter

Table II is a measure of the effect of single polymer flocculants on the precipitation of enzyme broths with the UV absorbance measured at 550 microns. It is a comparative table showing that, although Products A and D individually may perform effectively with regard to compaction (% volume solids) or clarity, the flocculants alone fail to perform satisfactorily as regards both criteria.

EP 0 581 002 A2

## TABLE II

### EFFECT OF SINGLE POLYMER FLOCCULANTS ON THE FLOCCULATION OF ENZYME BROTH

| Example No. | Flocculant | g/l Flocculant | Centrifuge 5 Minutes % Volume Solids | Clarity of Supernatant 550 Microns |
|---|---|---|---|---|
| 13* | A | 20 | DNS** | – |
| 14* | A | 25 | DNS** | – |
| 15* | A | 30 | DNS** | – |
| 16* | A | 35 | 23 | 1.69 |
| 17* | A | 40 | 23 | 0.96 |
| 18* | A | 45 | 25 | 0.43 |
| 19* | D | 5 | 32 | – |
| 20* | D | 10 | 30 | 0.24 |
| 21* | D | 15 | 26 | 0.26 |
| 22* | D | 20 | 25 | 0.25 |
| 23* | D | 25 | 25 | 0.18 |
| 24* | D | 30 | 26 | 0.17 |

\* Comparative Example, not in accordance with the invention

\*\* DNS--did not settle

Table III reflects the unexpectedly superior results achieved when using blends of B and C polymers on an enzyme broth in accordance with the present invention. While not wishing to be limited to any particular theory, the results of Examples 1-6 demonstrate that a flocculant composition with a 50 to 50 ratio of B polymer to C polymer, when used in low dosages from 15 to 25 grams per liter of enzyme broth, produces a supernatant with improved clarity and compaction as compared to the supernatant produced with either

5

polymer B (Comparative Examples 7*-9*) or polymer C (Comparative Examples 10*-12*) alone.

TABLE III

IMPROVED COMPACTION AND CLARITY OF SUPERNATANT WITH POLYMER BLENDS

| Example No. | Flocculant | Ratio | g/l | Centrifuge 5 Minutes % Volume Solids | Clarity of Supernatant 660 Microns |
|---|---|---|---|---|---|
| 1 | B/C | 4:1 | 20 | 18 | 0.242 |
| 2 | B/C | 4:1 | 30 | 18 | 0.497 |
| 3 | B/C | 4:1 | 40 | 20 | 0.793 |
| 4 | B/C | 1:1 | 15 | 18 | 0.344 |
| 5 | B/C | 1:1 | 20 | 18 | 0.218 |
| 6 | B/C | 1:1 | 25 | 20 | 0.263 |

Table IV reflects the unexpectedly superior results achieved when using blends with various ratios of A and D polymers on an enzyme broth in accordance with the present invention. The data include observed compaction and absorbance at the indicated dosage. The results of Examples 7-24 demonstrate that a mixture of polymers A and D produces a synergistic effect that results in a supernatant with improved clarity and compaction as compared to the supernatant with either polymer A (Comparative Examples 13*-18*) or polymer D (Comparative Examples 19*-24*) alone.

TABLE IV

IMPROVED COMPACTION AND CLARITY OF SUPERNATANT WITH POLYMER BLENDS

| Example No. | Flocculant | Ratio | g/1 | Centrifuge 5 Minutes % Volume Solids | Clarity of Supernatant 550 Microns |
|---|---|---|---|---|---|
| 7 | A/D | 70:30 | 20 | 25 | – |
| 8 | A/D | 70:30 | 25 | 27 | 0.16 |
| 9 | A/D | 70:30 | 30 | 28 | 0.25 |
| 10 | A/D | 70:30 | 35 | 26 | 0.57 |
| 11 | A/D | 70:30 | 40 | 27 | 0.49 |
| 12 | A/D | 70:30 | 45 | 25 | 0.49 |
| 13 | A/D | 50:50 | 20 | 25 | 0.35 |
| 14 | A/D | 50:50 | 25 | 27 | 0.31 |
| 15 | A/D | 50:50 | 30 | 27 | 0.45 |
| 16 | A/D | 50:50 | 35 | 25 | 0.42 |
| 17 | A/D | 50:50 | 40 | 25 | 0.33 |
| 18 | A/D | 50:50 | 45 | 25 | 0.29 |
| 19 | A/D | 30:70 | 20 | 25 | – |
| 20 | A/D | 30:70 | 25 | 27 | 0.24 |
| 21 | A/D | 30:70 | 30 | 28 | 0.28 |
| 22 | A/D | 30:70 | 35 | 27 | 0.23 |
| 23 | A/D | 30:70 | 40 | 24 | 0.15 |
| 24 | A/D | 30:70 | 45 | 25 | 0.14 |

Table V shows the unexpectedly superior results achieved when using blends with various ratios of A and D polymers on an enzyme broth in accordance with the present invention. The data include clarity and compaction at the indicated dosage. The clarity is based on visual observation, and each sample is mixed by inverting the stoppered clinical tube fifteen (15) times. These results show that the preferred ratio for A to D polymers is from 30 to 70 parts per weight A to 70 to 30 parts per weight D with the most preferred ratio being 30 to 50 parts per weight A to 70 to 50 parts per weight D. The results of Examples 49-60 show that increasing the mixing of the enzyme broth and flocculant mixture does not have any noticeable effect on the clarity or compaction of the supernatant.

## TABLE V

### IMPROVED COMPACTION AND CLARITY OF SUPERNATANT WITH POLYMER BLENDS

| Example No. | Flocculant | Ratio | g/l | Mixing (No. of Shakes) | Centrifuge 5 Minutes % Volume Solids | Clarity of Supernatant (Visual Observation) |
|---|---|---|---|---|---|---|
| 25 | A/D | 30:70 | 25 | 10+5 | 42 | Good |
| 26 | A/D | 30:70 | 30 | 10+5 | 37 | Excellent |
| 27 | A/D | 30:70 | 35 | 10+5 | 37 | Good |
| 28 | A/D | 30:70 | 40 | 10+5 | 38 | Excellent |
| 29 | A/D | 30:70 | 45 | 10+5 | 35 | Good - Excellent |
| 30 | A/D | 30:70 | 50 | 10+5 | 36 | Excellent |
| 31 | A/D | 50:50 | 25 | 10+5 | 37 | Poor |
| 32 | A/D | 50:50 | 30 | 10+5 | 26 | Good |
| 33 | A/D | 50:50 | 35 | 10+5 | 39 | Good - Excellent |
| 34 | A/D | 50:50 | 40 | 10+5 | 33 | Excellent |
| 35 | A/D | 50:50 | 45 | 10+5 | 28 | Excellent |
| 36 | A/D | 50:50 | 50 | 10+5 | 30 | Excellent |
| 37 | A/D | 70:30 | 25 | 10+5 | 32 | Fair |
| 38 | A/D | 70:30 | 30 | 10+5 | 32 | Good - Excellent |
| 39 | A/D | 70:30 | 35 | 10+5 | 27 | Excellent |
| 40 | A/D | 70:30 | 40 | 10+5 | 30 | Good - Excellent |
| 41 | A/D | 70:30 | 45 | 10+5 | 29 | Good - Excellent |
| 42 | A/D | 70:30 | 50 | 10+5 | 31 | Good - Excellent |

TABLE V (CONTINUED)

| Example No. | Flocculant | Ratio | g/l | Mixing (No. of Shakes) | Centrifuge 5 Minutes % Volume Solids | Clarity of Supernatant (Visual Observation) |
|---|---|---|---|---|---|---|
| 43 | A/D | 90:10 | 25 | 10+5 | 27 | Excellent |
| 44 | A/D | 90:10 | 30 | 10+5 | 29 | Excellent |
| 45 | A/D | 90:10 | 35 | 10+5 | 28 | Good – Excellent |
| 46 | A/D | 90:10 | 40 | 10+5 | 27 | Good – Excellent |
| 47 | A/D | 90:10 | 45 | 10+5 | 29 | Excellent |
| 48 | A/D | 90:10 | 50 | 10+5 | 29 | Good & Excellent |
| 49 | A/D | 70:30 | 40 | 15 | 32 | Excellent |
| 50 | A/D | 70:30 | 40 | 30 | 28 | Excellent |
| 51 | A/D | 70:30 | 40 | 45 | 29 | Excellent |
| 52 | A/D | 70:30 | 45 | 15 | 31 | Good – Excellent |
| 53 | A/D | 70:30 | 45 | 30 | 31 | Excellent |
| 54 | A/D | 70:30 | 45 | 45 | 28 | Excellent |
| 55 | A/D | 90:10 | 30 | 15 | 26 | Excellent |
| 56 | A/D | 90:10 | 30 | 30 | 28 | Excellent |
| 57 | A/D | 90:10 | 30 | 45 | 27 | Excellent |
| 58 | A/D | 90:10 | 35 | 15 | 30 | Good – Excellent |
| 59 | A/D | 90:10 | 35 | 30 | 27 | Excellent |
| 60 | A/D | 90:10 | 35 | 45 | 27 | Excellent |

The above mentioned patents, publications, and Test Methods are incorporated herein by reference.

Many variations in the present invention will suggest themselves to those skilled in this art in light of the above, detailed description. For example, instead of dimethylamine, the polyamine can include units derived from methylamine, methanolamine or dimethanolamine. Instead of a Mannich homopolymer of acrylamide, a Mannich copolymer of acrylamide containing 5-50% of a copolymer can be used. Instead of ethylenediamine, the quaternary polyamine can be derived from ammonia or diethylenetriamine. All such obvious modifications are within the full intended scope of the appended claims.

**Claims**

1. A process for the flocculation of an aqueous enzyme broth which comprises (a) adding to said broth a flocculant comprising a blend of (1) a Mannich acrylamide polymer and (2) a quaternary polyamine having a molecular weight of at least about 25,000.

2. A process according to Claim 1 wherein the ratio of (1) to (2) ranges from about 99:1 to 1:99, by weight real polymer solids, respectively.

3. A process according to Claim 1 wherein (1) is a Mannich homopolymer of acrylamide.

4. A process according to Claim 1 wherein (1) is a Mannich copolymer of acrylamide containing 5-50% of a comonomer.

5. A process according to Claim 1 wherein (1) contains 25-100 mol percent of dimethylaminomethyl groups.

6. A process according to Claim 1 wherein the quaternary polyamine employed is produced from (i) a mono(or di-)alkylamine or a mono(or di-)alkanolamine and (ii) epihalohydrin and (iii) up to about 10 mole percent of an alkylenediamine or ammonia.

7. A process according to Claim 6 wherein the mono(or di-)alkylamine employed is monomethylamine or dimethylamine.

8. A process according to Claim 6 wherein the monoalkanolamine employed is monoethanolamine.

9. A process according to Claim 1 also including the steps of (b) mixing the enzyme broth and flocculant; and (c) precipitating the enzyme broth/flocculant mixture.

10. A process according to Claim 1 wherein (2) is a quaternary polyamine which is the reaction product of an epihalohydrin and an amine or a mixture of amines, said polyamine having a molecular weight in the range of from about 25,000 to about 350,000.

11. A process according to Claim 6 wherein the epihalohydrin is epichlorohydrin.

12. A process according to Claim 6 wherein the amine comprises a mixture of dimethylamine and ethylenediamine.

13. A process according to Claim 6 wherein quaternary polyamine (2) is the reaction product of about 50 mole percent of epichlorohydrin, about 49 mole percent of dimethylamine and about 1 mole percent of ethylenediamine, said polyamine having a molecular weight of from about 50,000 to about 300,000.

14. A process according to Claim 1 wherein the enzyme is a protease.

15. A process according to Claim 1 wherein from about 10 to about 100 grams per liter of broth of flocculant mixture is added.

16. A flocculant composition which improves the clarity and compaction of the supernatant of an aqueous enzyme broth when added thereto in an amount of from about 10 to about 100 grams per liter of broth, said flocculant composition comprising:
    (1) 1.0 to 99.0 parts by weight of a Mannich acrylamide polymer and
    (2) 99.0 to 1.0 parts by weight of a quaternary polyamine having a molecular weight of at least about 25,000.